# EUROPEAN PATENT APPLICATION

(11) **EP 2 226 044 A2**
(43) Date of publication of application: **08.09.2010**
(21) Application number: 10155319.6
(22) Date of filing: 03.03.2010
(51) Int. Cl.: A61F 13/14

(54) **Therapeutic compression garment**

(30) Priority: 04.03.2009 US 397486
(71) Applicant: Tactile Systems Technology, Inc., Minneapolis, Minnesota 55413 (US)
(72) Inventor: Waldridge, Irene A.,, Minnesota, 55379 (US)
(74) Representative: Kirschner, Klaus Dieter

(57) **Abstract**

A therapeutic compression garment is provided. The instant garment includes a unilateral torso structure, a biceps structure, and a tensioning structure. The unilateral torso structure comprises uniquely configured structure portions corresponding to front and rear panel forming elements. Contiguous perimeter segments of the uniquely configured structure portions delimit an armscye segment for the unilateral torso structure. The biceps structure is non-reversibly united, at a perimeter segment thereof, to the armscye segment of the unilateral torso structure. The tensioning structure selectively and reversibly unites portions of the unilateral torso structure.

## Description

The present invention generally relates to a therapeutic compression garment, more particularly, to a pneumatically actuated chambered therapeutic compression garment for adorning a portion of the torso, more particularly still, to an easily self-applied, body conforming, multi-chambered, unilateral pneumatic chest garment for the treatment lymphedema, other forms of edema, and chronic wounds.

The lymphatic system consists of lymph vessels, lymph nodes and lymphoid tissues. It is a secondary system within the circulatory system that removes waste, more particularly, the lymphatic system collects and filters interstitial fluid of the body.

Unlike the closed-loop blood circulatory system, the lymphatic system works according to a one-way principal. That is, the lymphatic system is intended to drain away lymph which continually escapes from the blood in small amounts.

The lymph is first collected at the lymph capillaries, which in turn drain into larger vessels. The lymph is pumped in and out of these vessels by movements of adjacent muscles and by contractions of the walls of the larger vessels. Foreign matter and bacteria are filtered at various lymph node groups after which the fluid empties into the venous portion of the blood system, mainly through the thoracic duct. A healthy person will drain one to two liters of lymph fluid through this duct every twenty-four hours. Without proper drainage into the duct, lymphedema (i.e., edema of the lymph) results.

Lymphedema is a chronic condition characterized by an accumulation of fluid in the body causing a painful, debilitating swelling or edema of the affected area, most remarkably, but hardly exclusively, the limbs and their extremities. The swelling causes pain, discomfort, disfigurement, and interference with wound healing and, if left untreated, can cause fibrosis (i.e., skin overlaying affected areas becomes thicker, and more turgid, with lymph fluid known to leak through breaks in the skin leading to serious, life-threatening infection).

Lymphedema may result from surgery after removing one or more lymph nodes, or as a result of radiation treatments. Moreover, primary (congenital) malformations, trauma or filariasis are known causes of lymphedema, or conditions which diminish lymphatic function to the point of corrective action.

In regard to treatment, consensus opinion supports the use of a combination of therapeutic techniques, categorically referred to as complete decongestive therapy (CDT). Such therapy comprises combinations of direct lymphatic massage, use of compression garments, or bandaging. It is generally understood that CDT offers initial, but difficult to sustain benefits.

After diagnosis and evaluation, multiple clinic visits over several weeks are required. Certified therapists and supporting clinicians deliver therapies such as manual lymphatic drainage (MLD), gradient compression bandaging, and medically prescribed compression garment use; provide meticulous skin care and counseling in connection to same; and, further provide education and instruction relative to therapeutic exercise, and self-MLD, among other things. Patient skill development is essential to maintain the size of one or more reduced healthy limbs, for example, in a subsequent home-care scenario. Moreover, complications and/or the loss of clinician supervised gains, e.g., increased limb size, fibrosis, infection (e.g., cellulitis), increased pain, reduced range of motion and potential limb dysfunction, are accompanied not only by the stated physical ramification or condition, but also implicate meaningful emotional and economic capital.

In as much as home management of lymphedema advantageously, and arguably necessarily includes self-MLD, a great number of afflicted individuals are unable to perform same, owing to limited mobility, or are unable to receive assistance in furtherance of same owing to a lack of an available caregiver. In lieu of self-MLD, commercially available intermittent pneumatic compression (IPC) devices and attendant therapeutic methods are know and widely practiced.

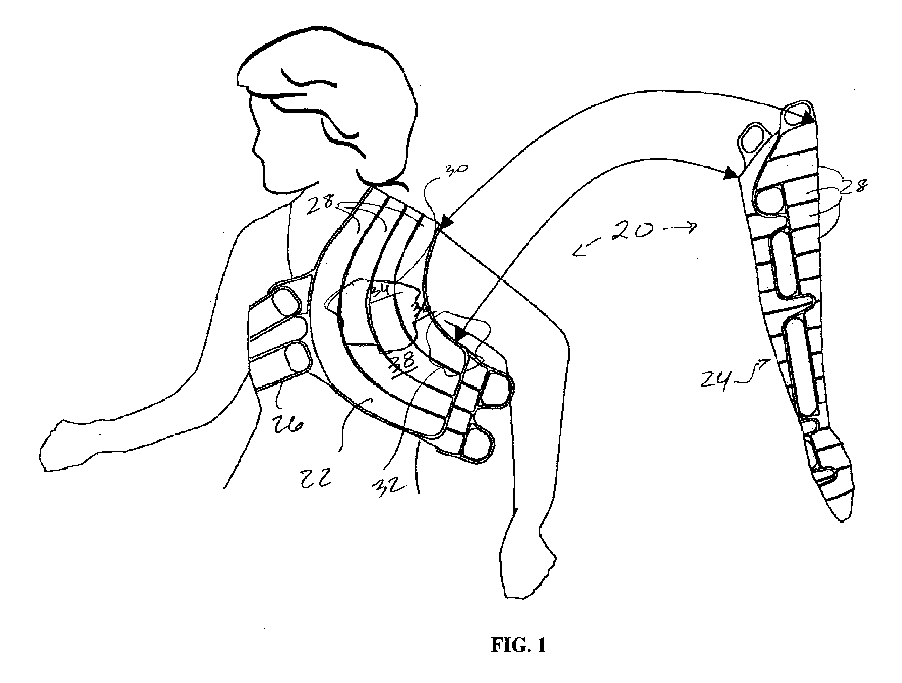

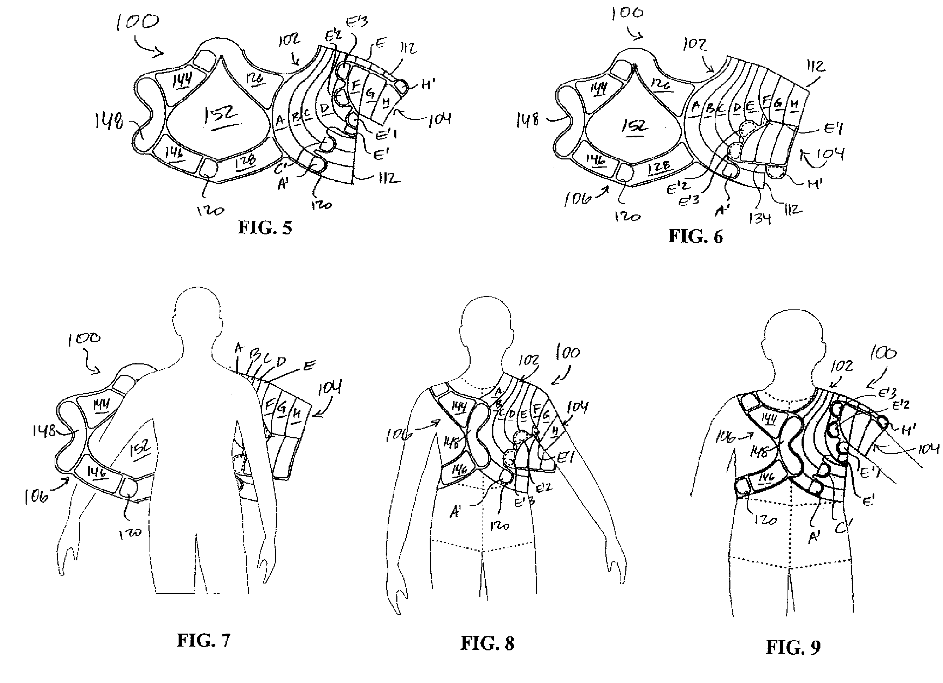

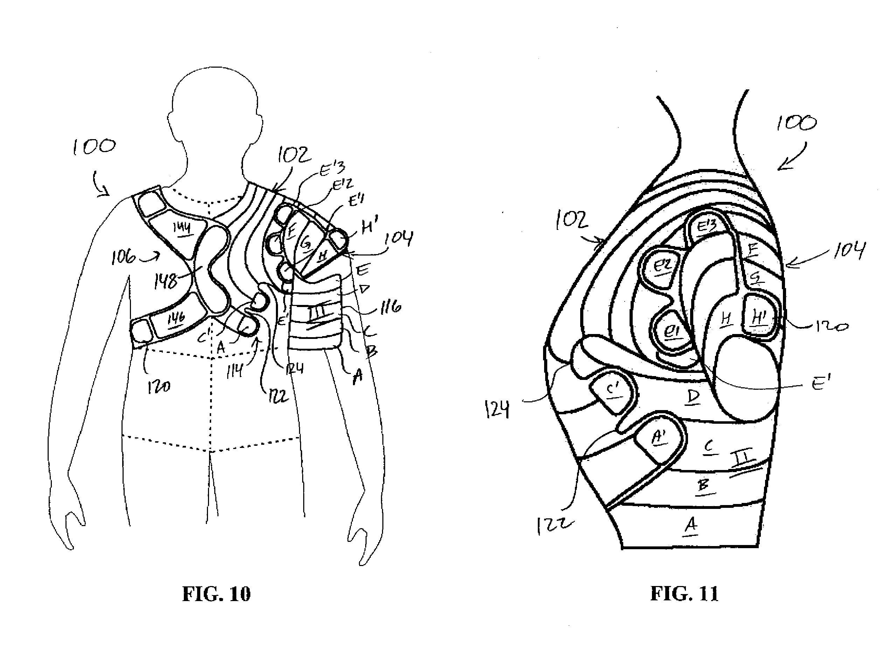

Beyond known jackets, vests, and pants from Lympha Press7 USA (NJ, USA), and limb structures from Bio Compression Systems, Inc. (NJ, USA), offerings of Applicant/assignee Tactile System Technology, Inc. (MN, USA) have been directed to therapeutic compression garment forming structures which, when applied in furtherance of treatment, contour the chambers or compartments of the structure around and about the body's natural curves (e.g., the trunk/torso and its characteristic landmarks such as the rib cage, pectoralis, deltoid, latisimus dorsi, and joints such as the elbow and wrist, and the hip). A heretofore known garment 20, characterized by unilateral torso structure 22, associated limb structure 24, and closure 26, of Applicant is shown with reference to FIG. 1, those structures and lymph drainage therapies, among other things, are described in, e.g., U.S. Pat. No. 6,860,862 B2, the complete disclosure of which is incorporated herein by reference in its entirety (see e.g., FIGS. 9 & 11 thereof).

In connection to the illustrative wrap structure of FIG. 1, a variety of shortcomings have been identified. More particularly, limitations fall into one of two primary areas, namely, those associated with "fit," and those associated with the "application" of the garment structure.

A "one-size fits most," let alone a "one-size fits all" approach appears unattainable with regard to the delivery of an especially effective lymphedema therapy via IPC therapy. For "off-the-shelf" unilateral torso garments, fit issues generally originate in connection to the chest and waist, namely proper circumscription of relatively small and large circumferenced chests/busts and/or waists.

Moreover, chambers or compartments 28 in the vicinity of the armscye of the structure (i.e., a fabric edge of the structure to which a sleeve is attached/affixed), as well as those in the vicinity of the breast/pectoralis, do not fully or completely follow the natural body contours in those areas; lacking is a three-dimensional fit about the breast/pectoralis 30 and/or the torso/limb transition area 32. As a matter of fact, the "flat" structure surfaces so formed about the torso, more particularly, the breast/pectoral area or region, create an undesirable, greater than intended pressure against the top of the breast 34, and, an undesirable, insufficient pressure in area 36 around the breast, and/or upon the rib cage 38 below the breast. Finally, while closure 26 of the FIG. 1 structure, namely, that extending down and away from the sternum with anchoring in the vicinity of the wearer's left shoulder blade (not visible), imparts tension upon the torso structure in furtherance of a snugging of the structure, absent is a complementary cross/diagonal pull from the sternum or between the breasts/pectoralis to create an improved snug fit, particularly along an affected sideline and/or waist region.

With regard to "application," i.e., the ability of an afflicted individual to "suit-up," the FIG. 1 torso structure has proven challenging to "fasten," i.e., owing to hindered mobility due to a lack of strength, flexibility or other skill, the straps are not easily anchored in furtherance of attaining a sought after body-conforming fit. Needless to say, those garments stylized as jackets, vests, and pants are likewise problematic for stricken individuals to self-apply as they are especially cumbersome, and most often oversized. Moreover, to the extent that the torso garment is suitably applied, integration of a limb structure presents it's own challenge, namely, it has proven difficult for many patients to apply the limb structure because it cannot be readily pulled incrementally toward the shoulder for attachment with the armscye of the torso structure.

In as much as able-bodied individuals might find the application of heretofore known garment structures straight forward, and not particularly taxing, lymphedema patients are not so situated. In light of the foregoing, it is believed especially desirable to minimize and/or eliminate barriers to effective home self-MLD therapy via the provision of more effective garment or wrap structures which enhance or fortify heretofore known IPC therapies. Moreover, it remains desirable to provide readily alterable/adjustable garments to "fit" a range of given or select body sizes, more particularly, garments which are easily and readily self-applied which are nonetheless effective pneumatic delivery articles. Furthermore, it is believed advantageous to provide a modified unilateral torso structure which more easily integrates with additional garment structures, e.g., a limb structure. Further still, it is believed advantageous to supply an improved tensioning structure for a unilateral torso structure.

A therapeutic compression garment is provided. Generally, the instant garment advantageously includes a unilateral torso structure, a biceps structure, and a tensioning structure. The unilateral torso structure comprises uniquely configured structure portions corresponding to front and rear panel forming elements. Contiguous perimeter segments of the uniquely configured structure portions delimit an armscye segment for the unilateral torso structure. The biceps structure is non-reversibly united, at a perimeter segment thereof, to the armscye segment of the unilateral torso structure. The tensioning structure selectively and reversibly unites portions of the unilateral torso structure.

As to advantageous particulars of the aforementioned garment structures, more particularly, but not necessarily, the unilateral torso forming structure comprises a multi-ply laminate torso panel characterized by a plurality of integral, discrete compartments. The compartments generally traverse free ends of the torso panel. The torso panel includes or is characterized by a portion intermediate its free ends which corresponds to a shoulder slope for the unilateral torso forming structure. The shoulder slope generally delimits uniquely configured front and rear multi-ply laminate torso panel portions, with the rear torso panel portion including shoulder and waist extensions.

As to the biceps structure, it too advantageously comprises a multi-ply laminate biceps panel characterized by a plurality of integral, discrete compartments. The compartments traverse free ends of the biceps panel. A peripheral portion of the biceps panel is non-reversibly united or affixed to the multi-ply laminate torso panel, more particularly, in a vicinity of the torso panel portion corresponding to the shoulder slope for/of the unilateral torso forming structure.

Finally, in connection to the tensioning structure for tensioningly uniting portions of the unilateral torso forming structure, it advantageously comprises a base panel, and shoulder and waist straps. The straps divergently extending from the base panel. The base panel is reversibly securable to the front multi-ply laminate torso panel portion so as to overlay a portion of a compartment adjacent a margin thereof. The shoulder strap is reversibly securable to the shoulder extension of the rear torso panel portion, while the waist strap is reversibly securable to the waist extension thereof.

Among other things, as will be later described and or detailed, the instant garment structures directly contribute to a unilateral therapeutic compression garment characterized by an integral yet fully adjustable upper limb component. Moreover, via a synergistic union of garment structures, torso landmarks are readily accounted for in an IPC garment, with such landmarks conformingly contoured about via easy self-application of the garment structures. Further still, via unique structure configurations, the synergistic union of garment structures for IPC therapy is enabled over a six-size range for a given set of garment structures.

More specific features and advantages obtained in view of those features will become apparent with reference to the drawing figures and the specification.
- FIG. 1: depicts structures of a known therapeutic compression garment, namely, a unilateral torso structure and associated limb structure as shown and described in Applicant's U.S. Pat. No. 6,860,862 B2;
- FIG. 2: depicts structures of the instant therapeutic compression garment, namely, a unilateral torso structure (center), a biceps structure (left), and a tensioning structure (right);
- FIG. 3: depicts the united structures of FIG. 2 in combination with a limb structure, namely, a limb structure extending from the biceps structure, the view being a "front" view;
- FIG. 4: depicts the united structures of FIG. 2, the view being a "rear" view; and,

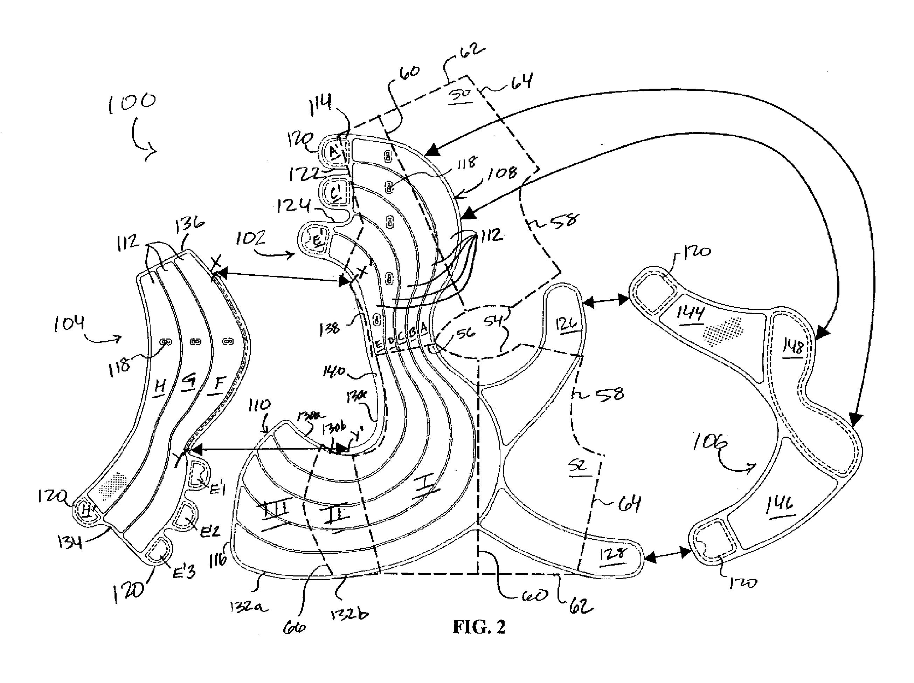

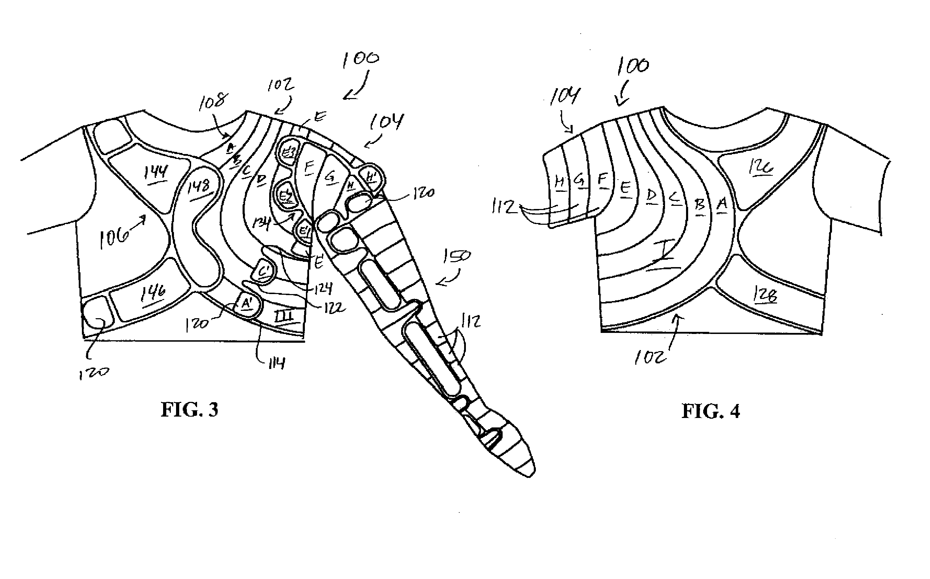

- FIGS. 5-11: depict interrelationships between and among the garment structures, e.g., disassociation of a portion of the tensioning structure (FIG. 5); "opening" of the sleeve of the biceps structure (FIG. 6); an initial self-application step (FIG. 7); alignment of the tensioning structure base and manipulation of the biceps structure so as to form a biceps sleeve about the upper arm (FIG. 8); "fit" adjustment of the biceps structure and sleeve thereby formed (FIG. 9); and, "fit" adjustment of the torso structure in furtherance of body conformity (FIGS. 10 & 11). The following description proceeds with general reference to FIGS. 2-11, and particular reference to FIGS. 2-4 on the one hand, and FIGS. 5-11 on the other hand. A presentation and discussion of the structures or elements of the improved therapeutic compression garment (FIG. 2) and the garment *per se* (FIGS. 3 & 4), precedes a discussion of the interrelationships between and among the garment structures in an application context (i.e., self-application of the garment by a patient). In advance of the subject detailed description of the invention and the drawings, several preliminary matters are noted.

First, in connection to fabrication preferences and/or the making/nature of the structures of the instant therapeutic compression garment, as well as advantageous "uses" thereof (i.e., articulated, specific therapeutic methods), Applicant's '862 patent, earlier referenced in connection to FIG. 1, with the disclosure thereof expressly incorporated herein by reference, provides non-limiting particulars. It is to be understood that the instant disclosure is primarily concerned with the garment structures per se, more particularly, their configuration, relationships and/or interrelationships in the context of the garment, namely, the synergistic combination of the structures in a unilateral chest garment which greatly aides performance of IPC therapy (i.e., improved delivery or execution of a home IPC therapy).

Second, as will become apparent with a presentation and discussion of the structures of FIG. 2, and garment(s) of FIGS. 3 & 4, the genesis of the instant garment, and thus the structures thereof, have origins in, among other things, a design objective wherein a single garment snugly "fits" a range of six "sizes." Owing to marketplace constraints/realities, it is not practical to develop, manufacture, and stock one garment per "standard body size" (see e.g. ASTM D5586, Standard Tables of Body Measurements for Women Aged 55 and older). Taking account for/of, among other things, torso related parameters such as bust, front waist length, waist circumference, shoulder length, chest circumference, neck base, shoulder slope, underarm to waist span, and armscye, some of which have front/back considerations or components, and appreciating that conventional apparel design/taibring techniques such as the use of pleats, darts, etc. are not meaningfully applicable to compression garments, especially in the context of a radio frequency (RF) welded garment, Applicant nonetheless proceeded in the face of the subject reality to produce the instant versatile garment/garment structures.

With general reference now to FIGS. 2-4, and particular reference, at least initially, to FIG. 2, there is shown the preferred structures or elements of the instant therapeutic compression garment. The garment 100 generally includes a unilateral torso structure 102, a biceps structure 104, and a tensioning structure 106. As will be later detailed, a perimeter segment of the biceps structure is non-reversibly united, joined or fastened to a perimeter segment of the torso structure as indicated by the right-left arrows intermediate those structures. Moreover, and as will be later detailed, the tensioning structure functions to tensioningly unite, selectively and reversibly, portions of the unilateral torso structure as indicated by the right-left arrows intermediate those structures.

As a visual aid in support of the unilateral torso structure of FIG. 2, torso patterns 50, 52, indicated or represented by the broken or dashed line, are shown overlaying same, the former being a "front" pattern and the latter a "rear" pattern. As a discussion aid in support of the unilateral torso structure, each of the patterns 50, 52 may be fairly characterized by a neck base 54, a common shoulder slope 56, an armscye 58, waist length 60, a waist circumference or "waistline" 62, and a sideline 64. Via comparison of the patterns, it is to be noted that the front pattern is less areally extensive than the rear pattern; a lateral extension or "wing" 66 extends from a sideline of the rear pattern 52 with a sideline of the front pattern 50 modified via the removal of an area delimited by the waistline, the "original" sideline, and the armscye. The area comprising rear pattern "wing" 66 substantially conforms to/with the absent area of front pattern 50; the excised front area has been essentially relocated to the rear pattern.

The unilateral torso structure 102 generally comprises uniquely configured structure portions corresponding to front 108 and rear 110 panel forming elements, namely, front and rear panel forming elements correlating to/with the aforementioned front 50 and rear 52 patterns. As shown, the unilateral torso structure 102 includes a plurality of integrally formed, discrete, non-overlapping chambers or compartments 112 which traverse the free ends 114,116 of the structure 102. Be that as it may, in as much as the compartment particulars are preferred and advantageous, they are not intended to be so limited, other known compartment/chamber/tubular arrangements being contemplated in the context of the instant structure. To facilitate further discussion, the compartments are sequentially labeled A-E in a direction progressing along the shoulder slope, from the neck base towards the armscye.

In as much as five compartments are depicted, and believed advantageous, compartments numbering within a range of about 4-8 for the torso structure may prove effective. Each compartment 112 is adapted for receipt of pressurized fluid, e.g., via the inclusion of a port 118 or the like for pneumatic discharge ingress, and may be fairly characterized as having a tapered region intermediate end portions thereof (i.e., compartments widths are at a relative "maximum" at their ends, and at a relative "minimum" at an intermediate region, namely, in the vicinity of the torso structure corresponding to the shoulder slope). Generally, and as indicated in connection to FIG. 2 and further appreciated with reference to FIGS. 3 & 4, at least a portion of compartment A of unilateral torso structure 102 is positioned or positionable so as to overlay the waist length (i.e., the sternum in the front and the spine in the rear), which substantially conforms to/with a lymphatic watershed of the lymphatic system.

As is readily appreciated with reference to FIG. 3, free ends 114, 116 (not visible but see FIG. 2) of the torso structure 102 are adapted for a reversible union, more particularly, a union characterized by a mating or matching of each of the compartments of the structure, i.e., one end portion of compartment A to the other end portion of compartment A, etc. In furtherance thereof, at least one of the free ends (e.g., free end 114) of torso structure 102 is equipped with closure structures or elements, e.g., tabs 120 as shown, or one or more strips, which are reversibly received upon or at the opposing free end, advantageously, a free end of front forming panel 108. A hook and loop fastening arrangement is contemplated in furtherance of engagement of the subject elements.

With regard to front forming panel 108 of torso structure 102, three spaced apart tabs delimit a free end of torso structure 102. The tabs, for the sake of discussion, are labeled A', C' & E' as each generally corresponds to compartments A, C & E of the torso structure. When suitably united with its corresponding "landing" in relation to rear forming panel 110 (FIG. 3), tabs A', C' & E' generally extend down-and-away from the torso mid-line or front waist length.

As is readily appreciated in connection to a review of FIG. 2, as compartments D & E are not coterminous with generally coterminating compartments A-C (i.e., the "ends" of compartments D & E extend beyond the generally coterminating ends of compartments A-C), tab E' likewise extends beyond generally coterminal tabs A' & C'. Moreover, in as much as there exists a general symmetry between and among tabs A' & C' relative to a uniform notch or gap 122 separating same, tab E' is spaced apart from tab C' via a non-uniform notch or gap 124. Via the afore described features of front forming panel 108, and as will become readily apparent in connection to a discussion of FIGS. 5-11 (note especially FIG. 11), an easily manipulatable, breast/pectoral conforming area is defined, whether such structure is present, or absent as result of a mastectomy.

With regard to rear forming panel 110 of torso structure 102, it may be fairly characterized by a series of regions or areas, namely, commencing from the shoulder slope of the torso structure and as indicated (FIG. 2), a scapula region I, a lateral or "wing" region II, and a terminal "rib" region III. Opposite the latter regions, shoulder 126 and waist 128 straps extend in up-and-out and down-and-out directions respectively, from rear forming panel 110 of unilateral torso structure 102, more particularly, from a peripheral margin of compartment A (i.e., the margin opposite the shared compartment A/B margin), advantageously, but not necessarily, from a rear waist line of the rear forming panel as indicated.

With regard to the regions of the rear forming panel of the torso structure, the former region (i.e., scapula region I) generally corresponds to the "left" torso area of rear pattern 52, as indicated in FIG. 2, note also FIG. 4. With regions II and III generally sequentially extending from region I (FIG. 2), "upper" margin segments 130a, 130b associated with those regions of the rear forming panel (i.e., the continuous margin associated or related to compartment E for regions II & III) extends up-and-out from an "upper" margin segment 130c of region I to a greater degree than extensions of an opposing "lower" margin segments 132a, 132b for regions II & III (i.e., the continuous margin associated or related to compartment A for regions II & III) in furtherance of aiding structure conformity about the armpit. Moreover, it should be appreciated that the depicted configuration for, and areal extent of the front rib region III of the rear forming panel, among other things, contributes to sizing flexibility for the instant therapeutic garment.

With continued reference to FIG. 2, biceps structure 104 includes a plurality of integrally formed, discrete, non-overlapping chambers or compartments 112 which traverse the free ends 134, 136 of the structure. Be that as it may, in as much as the compartment particulars are preferred and advantageous, they are not intended to be so limited, other known arrangements being contemplated in the context of the instant structure. To facilitate further discussion, and in keeping with the established convention of the instant disclosure, the compartments are sequentially labeled F-H in a direction progressing away from torso structure 102, more particularly, in a direction distal of the armscye segment thereof.

As previously noted or alluded to, biceps structure 104 is non-reversibly united, joined or otherwise physically connection at a perimeter segment thereof to a segment of the armscye of torso structure 102. More particularly, a segment X-Y along the peripheral margin of compartment F is affixed to armscye segment X'-Y' of the torso structure, a seam or the like thereby formed (FIGS. 3 & 4). The segment X'-Y' generally corresponds to/with a peripheral margin segment of compartment E, more particularly, adjacent compartment E peripheral margin segments 138,140 of the front 108 and rear 110 forming panels (i.e., those segments extending "away" from the shoulder slope of the torso structure). The X'-Y' segment extends from region I and terminates within region II of the rear panel forming element. With such arrangement, and as is readily appreciated in connection to FIG. 3 & 4, an adjustable biceps forming cuff/sleeve is enabled.

As is the case with the torso structure, a free end (e.g., free end 134) of the free ends of biceps structure 104 is adapted for selective, reversible union such that opposing end portions of compartments F-H register or overlay one another (see e.g., FIG. 3). In furtherance thereof, a tab, e.g., tab H', extends from a free end of compartment H, which, via a hook and loop interface, is readily receivable for retention upon a corresponding segment of an opposing end portion of compartment H. Moreover, a set of spaced apart tabs 120 extend from a periphery of biceps structure 104, more particularly, from and along a peripheral margin of compartment F (i.e., the margin opposite the shared compartment F/G margin) adjacent to segment X-Y thereof. Again, in keeping with the convention established herein, tabs E'1, E'2, and E'3, extending sequentially from segment X-Y, permit selective sleeve forming union with a portion of the front forming panel of the torso structure, more particularly, compartment E thereof (see e.g., FIG. 3). Via the so described elements, quick, easy adjustment about the upper limb, and establishment of a snug contoured fit is enabled.

In connection to compartments 112 of biceps structure 104, it should be readily appreciated with reference to FIG. 2 that the each compartment is successively "shorter" progressing distally from the armscye seam (i.e., progressing from compartment F to H). Among other advantages, it is believed that such configuration aides in a proper, conducive limb extension, namely, an approximate 30N angle between, among and/or for the limb and the torso.

With further continued reference to FIG. 2, tensioning structure 106 generally includes united straps (i.e., straps having a common origin), more particularly, shoulder 144 and waist 146 straps which extend or emanate from a base (i.e., sternum) panel 148. Adaptation of the free ends of straps 144, 146, via the inclusion of a tab or fastening element (e.g., a component of a hook and loop fastening system), permits reversible and adjustable union of the free ends of the shoulder and waist straps to their corresponding or counterpart elements extending from rear forming torso panel 110, namely, shoulder and waist straps 126 & 128. Likewise, the base panel 148 is so adapted for reversible receipt upon a portion of front forming panel 108, more particularly, a substantial portion of the base panel 148 is receivable for retention upon a segment of compartment A of torso structure 102. As is appreciated with reference to FIG. 3, an arcuate segment of the base panel 148 (i.e., the segment thereof adjacent straps 144, 146, or, to the extent that the panel may be fairly characterized as "goggle" shaped, the "upper" perimeter portion thereof), generally conforms with an arcuate peripheral margin segment of compartment A.

As will be shortly outlined in connection to self-application, the instant tensioning structure greatly improves the ability of an afflicted individual to adorn the garment. Moreover, owing to the tensioning structure configuration, the shoulder strap thereof pulls the torso structure diagonally up- and-across the chest, from the unaffected shoulder to and through the patient's centerline (i.e., front waist length) and further to the patient's affected waist area. Similarly, a complementary tension extends along the waist strap of the tensioning structure, more particularly, the torso structure is pulled diagonally down-and-across the chest in a direction from the affected shoulder, to and through the patient's centerline (i.e., front waist length) and further to the patient's unaffected waist area. Further still, the instant configuration is especially conducive to IPC therapy, as there exists a minimal contact/pressure area upon the non-affected torso portion (i.e., the unaffected area is not encumbered by unnecessary garment structure) with drainage "targets" easily reached, and, the subject arrangement permits the patient to easily and comfortably execute deep diaphragm breathing, a component of effective therapy.

Having to this point primarily described the garment structures and the resulting therapeutic compression garment formed thereby, self-application of the garment follows with reference to FIGS. 5-11. With regard to garment application, to the extent that further structural points and/or considerations arise, reference to prior figures are noted as applicable. Finally, in as much as the following self-application methodology is provided, it is intended as illustrative, not limiting.

Commencing from an associated condition for the garment structures 102, 104 and 106 of FIG. 2, for instance, the condition depicted in FIG. 3 absent limb structure 150 in combination therewith, the base panel 148 of tensioning structure 106 is released from its attachment to front panel 108 of torso structure 102 as shown in FIG. 5. Via the disassociation of base panel 148 from torso structure 102, a resultant stylized "drop" shaped aperture or opening 152 is formed, an opening delimited or bounded by the united shoulder 126 and waist 128 elements of both rear torso panel 110 and the corresponding straps 144, 146 of tensioning structure 106. Thereafter, biceps structure 104 is disassociated to its maximum extent via manipulation of closure tabs 120 thereof so as to "open" the sleeve defined by the "closed" structure. As should be readily appreciated, the sequence of the stated prepatory operations may be flip-flopped without consequence.

Having obtained the disassociated garment status of FIG. 6, self-application proceeds via receipt of an unaffected limb through drop-shaped opening 152 defined by tensioning structure 106, and the positioning/general alignment of the shoulder slope region of the torso structure upon the patient's shoulder slope. Thereafter, base panel 148 of tensioning structure 106 is reunited or reaffixed to front panel 108 of torso structure 102 so as to substantially overly a portion of compartment A as indicated in FIG. 8, with formation or reformation of the biceps sleeve about the affected limb via selective attachment of closure tabs 120 to corresponding portions, i.e., landings, of either the opposing structure end (i.e., tab H'), or front panel forming element 108 (i.e., tabs E'1-E'3) so as to generate the resulting configuration of FIG. 9. As previously noted, opposing ends of compartments A-E of the torso structure, and F-H of the biceps structure generally register or align (FIG. 3). At this application juncture, front rib region III of rear panel forming element 110 of torso structure 102 may be adjusted via realignment and reattachment (FIG. 10) so as to result in a snug contoured fit as depicted in FIG. 11, which further results from further adjustments in connection to tensioning the union of the torso structure portions 108, 110 via disassociation and refixing of either or both of shoulder 144/waist 146 straps of tensioning structure 106 to complete the self application process, and to the extent necessary, fine tune the structural relationships near/about limb torso interface, and/or breast/pectoral.

Having described the instant garment structures, garment so formed as well as a self-application sequence, there are nonetheless other variations/variants of the structures, garment, application sequence shown and/or described, some of which will become obvious to those skilled in the art. It is to be understood that this disclosure, in many respects, is only illustrative. Changes may be made in details, particularly in matters of shape, size, material, and arrangement of parts, as the case may be, without exceeding the scope of the invention. Accordingly, the scope of the subject invention is as defined in the language of the appended claims.

## Claims

1. A therapeutic compression garment comprising:
a. a unilateral torso structure comprising uniquely configured structure portions corresponding to front and rear panel forming elements, contiguous perimeter segments of said uniquely configured structure portions delimiting an armscye segment for said unilateral torso structure; and,
b. a biceps structure non-reversibly united, at a perimeter segment thereof, to said armscye segment.

2. The therapeutic compression garment of claim 1 further comprising a tensioning structure for selectively and reversibly uniting portions of said unilateral torso structure, preferably for selectively and reversibly uniting portions of said front and rear panel forming elements of said unilateral torso structure.

3. The therapeutic compression garment of claim 1 further comprising a tensioning structure **characterized by** a pair of united straps, free ends of the straps of said pair of united straps affixable to said rear panel forming element of said unilateral torso structure.

4. The therapeutic compression garment of claim 1 further comprising a tensioning structure for tensioning said torso structure, said tensioning structure comprising a sternum panel and shoulder and waist straps extending therefrom.

5. The therapeutic compression garment of claim 4 wherein said sternum panel is reversibly receivable upon a portion of said front panel forming element.

6. The therapeutic compression garment of claim 4 wherein free ends of said shoulder and waist straps are reversibly receivable upon a portion of said rear panel forming element.

7. The therapeutic compression garment of claim 1 wherein said unilateral torso structure includes a plurality of separately actuatable pneumatic chambers, or wherein said unilateral torso structure includes a plurality of discrete, non-overiapping compartments.

8. The therapeutic compression garment of claim 1 wherein said unilateral torso structure includes a plurality of discrete, non-overlapping compartments traversing free ends thereof.

9. The therapeutic compression garment of claim 1 wherein said biceps structure includes a plurality of separately actuatable pneumatic chambers, or
a plurality of discrete, non-overlapping compartments, or
a plurality of discrete, non-overlapping compartments traversing free ends thereof.

10. The therapeutic compression garment of claim 1 wherein said biceps structure includes a first peripheral segment adapted for reversible securement to said front panel forming element of said unilateral torso structure.

11. The therapeutic compression garment of claim 10 wherein said biceps structure includes a second peripheral segment, adjacent said first peripheral segment, adapted for reversible securement to an opposing peripheral segment of said biceps structure.

12. The therapeutic compression garment of claim 1 wherein said biceps structure includes plural fastening elements adjacent said perimeter segment non-reversibly united to said armscye segment.

13. The therapeutic compression garment of claim 1 wherein said rear panel forming element includes sequentially arranged scapula, lateral and front rib regions, wherein said front rib region preferably includes a free end of said rear forming panel.

14. The therapeutic compression garment of claim 13 wherein a free end of said front forming panel is reversibly receivable at said front rib region of said rear panel forming element, or wherein a free end of said front forming panel is matingly received at said front rib region of said rear panel forming element.

15. A therapeutic compression garment comprising:
a. a unilateral torso forming structure comprising a multi-ply laminate torso panel **characterized by** a plurality of integral, discrete compartments, said compartments traversing free ends of said torso panel, said torso panel including a portion intermediate said free ends corresponding to a shoulder slope for said unilateral torso forming structure, said shoulder slope delimiting uniquely configured front and rear mufti-ply laminate torso panel portions, said rear torso panel portions including shoulder and waist extensions;
b. a biceps structure comprising a multi-ply laminate biceps panel **characterized by** a plurality of integral, discrete compartments, said compartments traversing free ends of said biceps panel, a peripheral portion of said biceps panel non-reversibly united to said multi-ply laminate torso panel in a vicinity of said torso panel portion corresponding to the shoulder slope for said unilateral torso forming structure; and,
c. a tensioning structure for tensioningly uniting portions of said unilateral torso forming structure, said tensioning structure comprising a base panel, and shoulder and waist straps, said straps divergently extending from said base panel, said base panel reversibly securable to said front multi-ply laminate torso panel portion so as to overlay a portion of a compartment adjacent a margin of said front multi-ply laminate torso portion, said shoulder strap reversibly securable to said shoulder extension of said rear torso panel portion, said waist strap reversibly securable to said waist extension of said rear torso panel portion.
further comprising a tensioning structure for selectively and reversibly uniting portions of said unilateral torso structure, preferably for selectively and reversibly uniting portions of said front and rear panel forming elements of said unilateral torso structure.
